Europäisches Patentamt

(19) European Patent Office

Office européen des brevets

(11) Publication number: **0 331 361 B1**

## (12) EUROPEAN PATENT SPECIFICATION

(45) Date of publication of patent specification: **26.05.93**

(51) Int. Cl.⁵: **G01N 33/50**, G01N 33/566

(21) Application number: **89301816.8**

(22) Date of filing: **24.02.89**

(54) **Biologic test for susceptibility to alcoholism.**

(30) Priority: **29.02.88 US 161628**

(43) Date of publication of application:
**06.09.89 Bulletin 89/36**

(45) Publication of the grant of the patent:
**26.05.93 Bulletin 93/21**

(84) Designated Contracting States:
**AT BE CH DE ES FR GB GR IT LI LU NL SE**

(56) References cited:

PROCEEDINGS OF THE NATL. ACADEMY OF
SCIENCE USA, vol. 85, September 1988; L.E.
NAGY et al.; pp. 6973–6976&NUM;

ANNALS NEW YORK ACADEMY OF SCI-
ENCES, vol. 492 (Alcohol Cell), 1987; A.S.
GORDON, pp. 367–374&NUM;

PROCEEDINGS OF THE NATL. ACADEMY OF
SCIENCE USA, vol. 83, April 1986; A.S. GOR-
DON; pp. 2105–2108&NUM;

PROCEEDINGS OF THE NATL. ACADADEMY
OF SCIENCE USA, vol. 84, March 1987; I.
DIAMOND et al., pp. 1413–1416&NUM;

(73) Proprietor: **Diamond, Ivan F.**
**761 San Diego Road**
**Berkeley California 94707(US)**

Proprietor: **Gordon, Adrienne S.**
**2350 Hilgard Street**
**Berkeley California 94709(US)**

(72) Inventor: **Diamond, Ivan F.**
**761 San Diego Road**
**Berkeley California 94707(US)**
Inventor: **Gordon, Adrienne S.**
**2350 Hilgard Street**
**Berkeley California 94709(US)**

(74) Representative: **Harrison, David Christopher**
**et al**
**MEWBURN ELLIS 2 Cursitor Street**
**London EC4A 1BO (GB)**

**Description**

The present invention relates generally to assay methods for determining susceptibility toward alcohol‒ism. More particularly, the invention relates to a method for measuring a cellular indicator within a patient sample and relating the measurement to the patient's susceptibility to alcoholism.

Alcoholism is a world‒wide public health and socioeconomic problem. Unfortunately, at present little is understood concerning the biochemical causes and effects of alcoholism, and there exists no accepted biologic test for determining a patient's susceptibility to alcoholism.

It would be desirable to provide a specific biologic test which can identify alcoholism susceptibility in a patient. Such a test should be simple, rapid, and accurate, even when the patient suffers from medical complications, including liver disease. Such individuals could then be recognized early and appropriate counselling and/or medical treatment might be able to prevent the subsequent development of alcoholism

Gordon et al. (1986) Proc. Natl. Acad. Sci. USA 83: 2105‒2108 describes that ethanol acutely increases adenosine receptor‒stimulated cAMP levels in a clonal neural cell line, and that with time the cells adapted to the presence of ethanol, showing a reduction in adenosine receptor‒stimulated cAMP levels. Tabakoff et al. (1988) N. Engl. J. Med. 318:134‒139 teaches that the inhibition of monoamine oxidase by ethanol is significantly higher in the platelets of alcoholics than in those of controls and that stimulation of platelet adenylate cyclase activity by guanine nucleotide, cesium fluoride, and prostaglandin $E_1$ is significantly reduced in the platelets of alcoholics. A portion of the work underlying this application was presented in Diamond et al. (1987) Proc. Natl. Acad. Sci. USA 84: 1413‒1416 and in Gordon et al. (1987) New York Acad. Sci. 492: 367‒373.

All this prior art was however directed at determining actual alcoholism.

According to the invention alcoholism susceptibility in a patent is determined by measuring a cellular indicator which correlates with intracellular cAMP synthesis. Substantial deviation of the measured value of the cellular indicator from an expected normal value is diagnostic of alcohol susceptibility. Conveniently, the cellular indicator may be the intracellular cAMP concentration, where an increase, for example of 50%, in stimulated cAMP concentrated in cells cultured for at least three divisions in the absence of alcohol is diagnostic of alcohol susceptibility. Alternatively, cells from persons susceptible to alcohol dependence cultured for at least three divisions in the absence of ethanol followed by a predetermined exposure to ethanol will show a reduction in stimulated cAMP production, for example of 25% or more. Other cellular indicators which are characteristic of intracellular cAMP synthesis, however, may also find use.

In the exemplary embodiment, the basal and/or receptor‒stimulated cAMP levels are determined by immunoassay in cultured lymphocytes. Stimulated cAMP production is increased in cultured lymphocytes derived from patients at risk from alcoholism, while basal cAMP production is substantially the same for all patients. In contrast, cultured lymphocytes from susceptible subjects exposed to ethanol will display a reduction in stimulated cAMP production while basal production remains substantially unchanged.

In the following, some material is repeated from the Diamond et al. and Gordon et al. (1987) papers cited above, to illustrate the present invention by way of comparison.

Fig. 1 presents basal and stimulated cAMP levels in lymphocytes from alcoholics (open bars) and control subjects (solid bars). Phenylisopropyladenosine (PIA) is an adenosine receptor agonist. Each bar represents the mean ± SEM (n = 10 for basal and PIA; n = 9 for PIA plus ethanol).

Fig. 2 presents basal and PIA‒stimulated cAMP levels in T cells from alcoholics (open bars) and control subjects (solid bars). Each bar represents the mean ± SEM (n = 6).

Fig. 3 represents in the means ± SEM for basal and stimulated (0.1 mM PIA) levels of cAMP. No adenosine deaminase (n = 4), 0.02 U/ml (n = 6) and 0.5 U/ml adenosine deaminase (n = 4). Addition of ADA significantly (p<0.04) increased stimulated levels of cAMP but did not affect basal values.

Fig. 4. Lymphocytes were grown in fresh cell culture media for 8 days. Fresh media was added 24 hours or 48 hours prior to assay. Bars represent means ± SEM for cAMP levels under basal and stimulated conditions (0.1 mM PIA). Addition of fresh media significantly (p<0.01) increased stimulated levels of cAMP but did not affect basal values.

Fig. 5. Lymphocytes were grown in culture for 8 days. 200 mM ethanol was added for the last 48 hours, and adenosine receptor‒stimulated (0.1 mM PIA and 0.1 mM PIA with 150 mM ethanol) cAMP levels were determined. Bars represent means ± SEM from 6 experiments. Chronic ethanol treatment significantly (p<0.05) reduced basal and stimulated levels of cAMP.

Fig. 6. Lymphocytes were grown in culture for 7 days in the absence of ethanol and assayed for adenosine receptor‒stimulated cAMP levels. Bars represent means ± SEM from 7 experiments. Values for alcoholics were significantly (p<0.001) greater than non‒alcoholics.

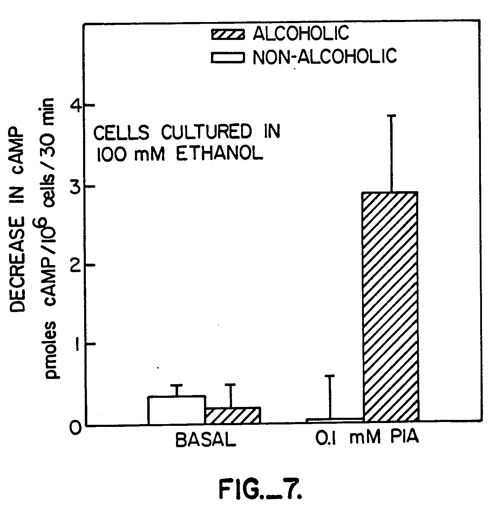

Fig. 7. Lymphocytes were grown in culture for 7 days. 100 mM ethanol was added for the last 24 hours, and adenosine receptor − stimulated cAMP levels determined. Values represent the mean ± SEM of the difference between cells grown in the absence of ethanol and cells chronically exposed to ethanol. Adenosine receptor − stimulated cAMP levels were decreased to a significantly greater degree in alcoholics (n = 7) than non − alcoholics (n = 9) p<0.003). There was no difference in basal values.

The present invention provides a simple and reliable test for determining alcoholism susceptibility in individual patients. The test relies on a biologic assay of a patient sample and is highly accurate regardless of patient complications, such as liver disease.

For the purposes of the present invention, alcoholism susceptibility is defined as a genetic predisposi − tion toward alcohol dependence.

The test of the present invention as set forth in the claims relies on measuring a cellular indicator which is related to the regulation of intracellular adenosine 3',5' − monophosphate (cyclic AMP or cAMP) synthesis. The most common cellular indicator will be intracellular cAMP concentration measured in a cellular sample. Whereas cAMP concentrations below normal in fresh cells are diagnostic of alcoholism, in contrast, cAMP concentrations above normal in cells cultured for three divisions in the absence of ethanol or below normal in cells exposed to ethanol are diagnostic of alcoholism susceptibility. It will be appreciated by those skilled in the art, however, that a number of other cellular indicators are available which can additionally be related to cAMP synthesis and which may be diagnostic of alcoholism susceptibility. Such additional cellular indicators and methods for their determination are discussed in greater detail hereinbelow.

The tests of the present invention may be performed on a variety of patient samples, with the particular type of sample depending primarily on the nature of the cellular indicator which is to be measured. Suitable patient samples include both cellular samples and fluid samples. Cellular samples may be derived from tissue, e.g., skin fibroblasts, or may be circulating cells, e.g., lymphocytes, monocytes, eosiphils, basophils, platelets, erythrocytes, polymorphonuclear lymphocytes, and the like. Fluid samples will include blood, plasma, serum, cerebral fluid, tears, saliva, semen, sputum, and the like. The collection and initial preparation of such samples are well described in the patent and scientific literature.

In the exemplary embodiment, cAMP concentration is measured in freshly isolated or cultured patient lymphocytes, including T − cells, B − cells, or both. The lymphocytes may be isolated from blood or lymph fluid by conventional techniques, typically by differential centrifugation, and (if desired) may then be cultured in a serum − free defined media which eliminates variability resulting from serum components. A particular defined media suitable for lymphocyte growth is described in the Experimental section hereinafter. Lymphocytes are seeded into the growth media at a density in the range from about 0.1 to 0.15 x $10^6$ cells/ml and are maintained as suspension cultures at 37˚C under humidified 5% $CO_2$. In addition to the normal components for maintaining the lymphocytes, the culture media may also include a phosphodiesterase inhibitor, an adenosine receptor agonist or other hormone receptor agonists. Such additional components may be added to the growth media initially or after a predetermined time period.

Intracellular cAMP concentration may be measured in fresh or cultured lymphocytes as follows. The lymphocytes are placed in fresh defined medium, such as Dulbecco's modified phosphate buffered saline containing 25 mM Hepes and 10 mM glucose. A phosphodiesterase inhibitor, such as ZK62711 from Rolipram, is added at a suitable concentration, typically about $10^{-5}$ M, and the cells incubated at 37˚C for several minutes. An enzyme capable of degrading adenosine, typically adenosine deaminase (ADA), is also added at a suitable concentration, typically about 1 U/ml, to eliminate the stimulatory effect of released adenosine during the assay. Suitable agonists, such as phenylisopropyladenosine (PIA) at concentrations at from $10^{-4}$ to $10^{-9}$ M, ethanol at concentrations of about 25 to 200 mM, or both, are added to the media and the cells allowed to incubate for a predetermined period, typically about 30 minutes. Incubation is stopped by disrupting the cells, typically by adding a suitable detergent and acid, such as 2% of a nonionic detergent and 0.1 N HCl, and extracting the cells on ice. cAMP concentration may then be measured in a centrifugation supernatant by radioimmunoassay. Stimulated cAMP concentrations are suitable as a basis for performing the test of the present invention.

Comparative:

A reduction in cAMP concentration of at least about 50% of the normal basal or normal stimulated concentrations in freshly isolated lymphocytes will be considered diagnostic of alcoholism. The expected cAMP concentration in alcoholic patients will be even lower than the diagnostic level, typically being about 25% of the corresponding normal value, or less. The normal, diagnostic, and expected cAMP concentra − tions for basal and stimulated (agonist or agonist and ethanol) intracellular cAMP synthesis are as follows:

| cAMP Concentration | Measured Value* | | |
|---|---|---|---|
| | Normal | Diagnostic | Expected |
| Basal | 10 ± 2 | 5 | 2 ± 1 |
| Stimulated (Agonist) | 15 ± 3 | 7.5 | 4 ± 1 |
| Stimulated (Agonist and Ethanol) | 25 ± 5 | 12.5 | 5 ± 1 |

\* All values in pmol/$10^6$ cells.

While both basal and stimulated cAMP concentrations are useful, it will normally be preferred to measure cAMP concentration from stimulated lymphocyte samples since the absolute difference between the normal cAMP concentration and the diagnostic and expected cAMP concentrations is substantially greater. In particular, the difference is greatest in samples which have been stimulated with both an adenosine receptor agonist and alcohol. The greater difference provides for a higher reliability in the test.

Performance of the cAMP assay on fresh lymphocytes (i.e., those separated from blood or lymph fluid and not cultured prior to the assay) is diagnostic of the patient's present status as an alcoholic. Assay results demonstrating intracellular cAMP concentrations reduced below the diagnostic values set forth above indicate a present alcohol dependence in the subject. Such results, however, do not necessarily indicate a genetic predisposition toward alcohol dependence.

EXAMPLE:

To determine a genetic predisposition toward alcohol dependence (in either alcoholic or non-alcoholic patients), it is necessary to culture the patient's lymphocyte sample in the absence of alcohol for several cell divisions, usually for a period of at least about 6 days, prior to performing the cAMP assay. After such a period, the culture is composed primarily of daughter cells which have never been exposed to alcohol. The cells of alcoholics will display an increased intracellular cAMP concentration in the presence of stimulation, either an adenosine agonist or an adenosine agonist and ethanol. The concentration increase will be at least 50%, and will usually be 100% or greater. The basal cAMP concentration, however, will not be substantially changed. The normal, diagnostic, and expected cAMP concentrations for basal and stimulated (agonist or agonist and ethanol) intracellular cAMP synthesis in lymphocyte culture are as follows:

| cAMP Concentration | Measured Value* | | |
|---|---|---|---|
| | Normal | Diagnostic | Expected |
| Basal | 2 ± 1 | N/A | 2 ± 1 |
| Stimulated (Agonist) | 3 ± 1 | 5 | 8 ± 2 |
| Stimulated (Agonist and Ethanol) | 4 ± 1 | 8 | 12 ± 2 |

\* All values in pmol/$10^6$ cells.

The difference in the normal and diagnostic values of cAMP concentration set forth above for the determination of alcoholism susceptibility may be greatly amplified by exposing the lymphocyte culture to a low concentration of ethanol, typically about 100 mM, during the final portion of the culture period, typically for about the last 24 hours. Such exposure results in the substantial depression of stimulated cAMP synthesis in the lymphocytes of alcoholic patients (typically at least about 25% of the expected normal value, more typically being at least about 30%, and frequently being 40% or greater) while leaving the corresponding value for non-alcoholic patients substantially unchanged.

In addition to measurement of intracellular cAMP concentration, as just described, measurement of a number of additional cellular indicators may also serve to discriminate between susceptible and non-susceptible patients according to the present invention.

GENERAL DISCUSSION:

Materials and Methods

Description of Patients

Ten volunteer, actively drinking alcoholic patients were matched for age and sex with 10 normal individuals (9 men and 1 woman). Each alcoholic was also age‒matched with a nonalcoholic‒outpatient with liver disease. The liver disease group was not sex‒matched (5 men and 5 women). Estimates of lifetime ethanol consumption were based on medical chart review and a patient questionnaire from which ethanol consumption was derived by the formula: lifetime ethanol consumption (kg) = [(grams of ethanol per serving) x (servings per day) x (days per month drinking) x (12) x (years of drinking)] ÷ 1000. Blood ethanol levels were measured at the time blood was drawn for the lymphocyte cAMP studies.

Materials

Heparinized Vacutainers were obtained from Becton Dickinson. Histopaque‒1077 and RIA‒grade bovine serum albumin were obtained from Sigma. $N^6‒(R‒$phenylisopropyl)adenosine (PIA) and fatty acid‒free bovine serum albumin were obtained from Boehringer Mannheim. Ro 20‒1724 was from Hoffmann‒La Roche. Rabbit antiserum raised against cAMP conjugated to bovine serum albumin was obtained from Miles. $^{125}$I‒labeled cAMP was provided by Chemicon (Los Angeles). Sheep whole blood in Alsever's solution was obtained from Hana Media (Berkeley, CA). Cell‒culture‒grade 100 x 20‒mm plastic Petri dishes and 12 x 75‒mm polypropylene tubes were obtained from Falcon.

Cell Preparation

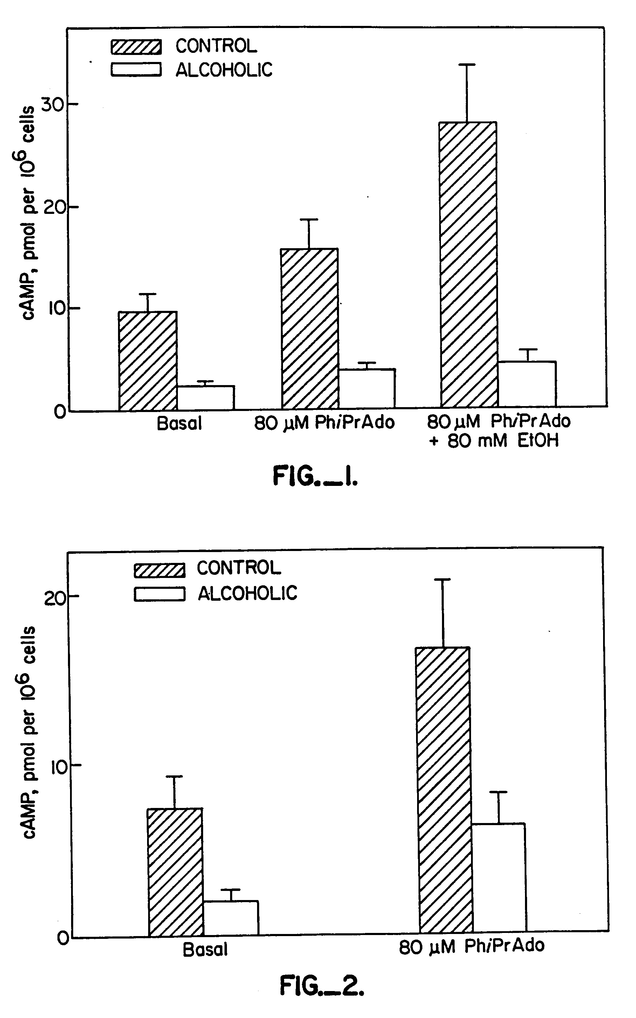

Human lymphocytes were prepared by established procedures (Boyum (1968) J. Clin. Lab. Invest. 21 (Suppl. 97):77‒89). Peripheral blood was collected in heparinized glass tubes (143 USP units/10 ml). Platelet contamination was reduced in the cell preparation by centrifuging 30‒ml aliquots of blood for 20 min. at 100 x $g$ and removing the upper, platelet‒rich layer. The remaining blood was diluted 1:3 with calcium/magnesium‒free Hanks' balanced salt solution (HBSS) containing 25 mM Hepes (pH 7.2). Ten milliliters of Ficoll (density = 1.077 g/cm$^3$) were layered under 40 ml of the diluted blood in a 50‒ml centrifuge tube. After a 25‒min centrifugation at 400 x $g$ at room temperature, the mononuclear cells, including B and T lymphocytes and monocytes, were removed from the Ficoll/plasma interface with a glass pipette. The cells were washed twice in HBSS and suspended at a concentration of 10$^6$ cells per ml in Dulbecco's phosphate‒buffered saline (DPBS) containing 0.2% glucose and 25 mM Hepes (pH 7.2). Cell viability, assessed by trypan blue exclusion, averaged 95%. This mixed‒lymphocyte preparation was used in all studies except for experiments with T cells described in Fig. 2.

Isolation of T and B Cells

T cells were separated by erythrocyte (E)‒rosette formation, using a modification of the method of Kasakua et al. (1983) J. Immunol. 131:2307‒2315. In order to isolate T cells, the heterogeneous lymphocyte preparation was resuspended after the final wash in HBSS to a concentration of 5‒10 x 10$^6$ cells per ml in sheep erythrocyte‒absorbed DPBS containing 0.2% glucose and 5% bovine serum albumin (fatty acid‒free). The DPBS solution was also used to prepare a 3% suspension of washed, packed sheep erythrocytes. Eight milliliters of both cell suspensions were mixed in a plastic centrifuge tube and kept at room temperature for 10 min. Ten milliliters of Ficoll was then layered under the mixture and the tubes were centrifuged for 30 min at 400 x $g$ at room temperature. Rosettes (E$^+$) of human T lymphocytes and sheep erythrocytes formed a pellet at the bottom of the tube, while nonrosetting (E$^-$) B cells and monocytes collected at the Ficoll interface. Erythrocytes in the E$^+$ pellet were lysed with lysing reagent (Ortho Diagnostics) and the remaining T cells were washed twice with calcium/magnesium‒free DPBS and resuspended at 10$^6$ cells per ml in RPMI‒1640 medium supplemented with 2% bovine serum albumin, 2 mM L‒glutamine, antibiotics (50 units of penicillin per ml and 50 $\mu$g of streptomycin per ml), and 25 mM Hepes (pH 7.2). T cells from alcoholics and control subjects were carried through all preparations and analytical procedures simultaneously.

B cells and monocytes were removed from the Ficoll interface, washed twice with calcium/magnesium‒free DPBS, and resuspended at a concentration of 10$^6$ cells per ml in supplemented RPMI – 1640 without Hepes. This cell suspension was then poured into plastic Petri dishes (10 ml per dish) and held for 120 min at 37°C in a humidified 7% $CO_2$ incubator. Monocytes adhered to the dish, and B cells in the suspension were removed and washed twice with DPBS, resuspended at $10^6$ cells per ml in supplemented RPMI – 1640, and kept at 4°C.

Both B – and T – cell suspensions were incubated with 7.3 mM L – leucine methyl ester for 45 min at room temperature to eliminate contaminating monocytes prior to use (Thiele et al. (1983) J. Immunol. 131:2282 – 2290). The reaction was stopped by the addition of 1 ml of fetal bovine serum. The cells were washed twice with DPBS and suspended at a concentration of $10^6$ cells per ml in DPBS containing 0.2% glucose and 25 mM Hepes (pH 7.2).

Assay for Basal and Stimulated cAMP in Cells

Cells (5 x $10^5$ in 0.5 ml) were preincubated in triplicate in 12 x 75 – mm polypropylene tubes for 5 min with 90 $\mu$M Ro 20 – 1724, a phosphodiesterase inhibitor, and then incubated for 60 min with or without 80 $\mu$M PIA, an adenosine receptor agonist, or 80 mM EtOH in a final volume of 0.6 ml. The incubation was stopped by adding 50 $\mu$l of 2% Nonidet P – 40 in 1 M HCl and extracting the cells on ice for 10 min. cAMP levels were determined in a 700 x *g* supernatant by radioimmunoassay (Koch et al. (1983) Biochem, Biophys. Res. Commun. 131:2282 – 2290). cAMP concentrations were determined without investigator bias by using an automated nonlinear regression analysis procedure on a Beckman DP5500 gamma counter. Protein was measured as described by Lowry et al. (1951) J. Biol. Chem. 193:265 – 275, using bovine serum albumin as standard. Standard conditions for cAMP determination were first established using 31 preparations of lymphocytes from normal volunteers.

RESULTS

Each of the alcoholic patients had normal weight for height, based on the Metropolitan Life Insurance height/weight table, and each individual was considered to be well – nourished by an experienced clinician, who examined all of the patients in this study. Five alcoholics had normal neurologic examinations. The other five alcoholic patients had mild memory deficits (3), mild peripheral neuropathy (2), and mild cerebellar gait ataxia (2). Diagnoses in the liver disease group included hemochromatosis (2), chronic active hepatitis (2), postnecrotic cirrhosis, metastatic carcinoma (breast), viral hepatitis, "autoimmune" disease with hepatitis, idiopathic elevation of liver enzymes, and cryptogenic cirrhosis. The mean lifetime consumption of ethanol in the alcoholic group was 13 times greater than in the control group and 43 times greater than in the liver group. Two alcoholics in the study had blood ethanol levels of 1 and 2.5 mg/ml, respectively, while the remaining patients had no detectable blood ethanol. There was no evidence of malnutrition in the alcoholic subjects. Their hemoglobin levels and mean corpuscular volumes were normal, indicating adequate iron and folic acid ingestion. Transketolase activity was normal, suggesting an adequate thiamine – containing diet, and such markers for malnutrition as serum albumin and lymphocyte counts were also normal. Alcoholics and patients with liver disease exhibited abnormalities of some liver enzymes, but there were no statistically significant differences between the alcoholic and matched liver disease groups. Laboratory values in alcoholic patients were similar to normal controls except for significantly higher serum glutamic – oxaloacetic transaminase and globulin.

PIA is an adenosine receptor agonist that is not readily transported into cells (Daly et al. (1981) Life Sci. 28:2083 – 2097). Under standard conditions of assay, unstimulated (basal) and PIA – stimulated levels of cAMP in normal human lymphocyte preparations (n = 31) were 8.97 ± 0.88 (SEM) and 14.85 ± 1.37 pmol per $10^6$ cells, respectively. PIA – stimulated cAMP accumulation varied as a linear function of cell number, and ethanol added in vitro further increased PIA – stimulated cAMP levels in a concentration – dependent manner without changing basal cAMP levels (data not shown). There was no correlation of basal or stimulated cAMP levels with age or sex, but day – to – day variation was noted in some subjects.

Alcoholics showed highly significant depressions of basal and PIA – stimulated cAMP levels in intact lymphocytes when compared to normal subjects (Fig. 1) or patients with liver disease. *P* values were calculated by Wilcoxon rank sum test comparing controls to alcoholics and alcoholics to patients with liver disease. Mean basal cAMP levels (pmol per $10^6$ cells) in controls, alcoholics, and patients with liver disease were 9.55 ± 1.65 (SEM), 2.30 ± 0.34 (*P* = 0.0004), and 8.33 ± 1.29 (*P* = 0.0005), respectively. Mean PIA – stimulated cAMP levels were 15.81 ± 2.52, 3.72 ± 0.53 (P = 0.0002), and 14.04 ± 1.93 (*P* = 0.0005), respectively. The difference between alcoholics and controls was also striking when the effect of 80 mM ethanol on PIA – stimulated cAMP levels was compared (Fig. 1). In addition, the percent response to ethanol was decreased markedly in lymphocytes from alcoholics compared to cells from normal subjects. Ethanol increased PIA − stimulated cAMP levels by 17.0% ± 3.8 (SEM) in lymphocytes from alcoholics compared to 71.0 ± 9.6% in normal individuals ($P$ = 0.002). The mean cAMP level in PIA − stimulated lymphocytes from alcoholics after addition of ethanol was 4.33 ± 0.96 (SEM) pmol per $10^6$ cells, while the value for normal controls was 28.0 ± 5.2.

Most of the adenosine receptors in lymphocytes appear to be associated with the T − cell fraction (Moroz et al. (1981) Immunol. Immunopath. 18:47 − 53), although this is controversial (Bonnafous (1981 − 1982) J. Recept. Res. 2:347 − 366). It is possible that a reduction in the percentage of circulating T cells in alcoholics might account for the reduced cAMP levels in alcoholics. To confirm that these reduced levels of stimulated cAMP in cells of alcoholics were not due to changes in the lymphocyte population, T cells from unmatched normal and alcoholic subjects were isolated and assayed separately. Fig. 2 shows that there was also a marked reduction in basal ($P$<0.02) and PIA − stimulated cAMP levels ($P$<0.03) in T cells recovered from alcoholic patients compared to controls. We found no significant effect of PIA on cAMP accumulation in B cells from alcoholics or controls (data not shown). Basal levels of cAMP were similar in B and T cells from each individual alcoholic or control subject studied (data not shown).

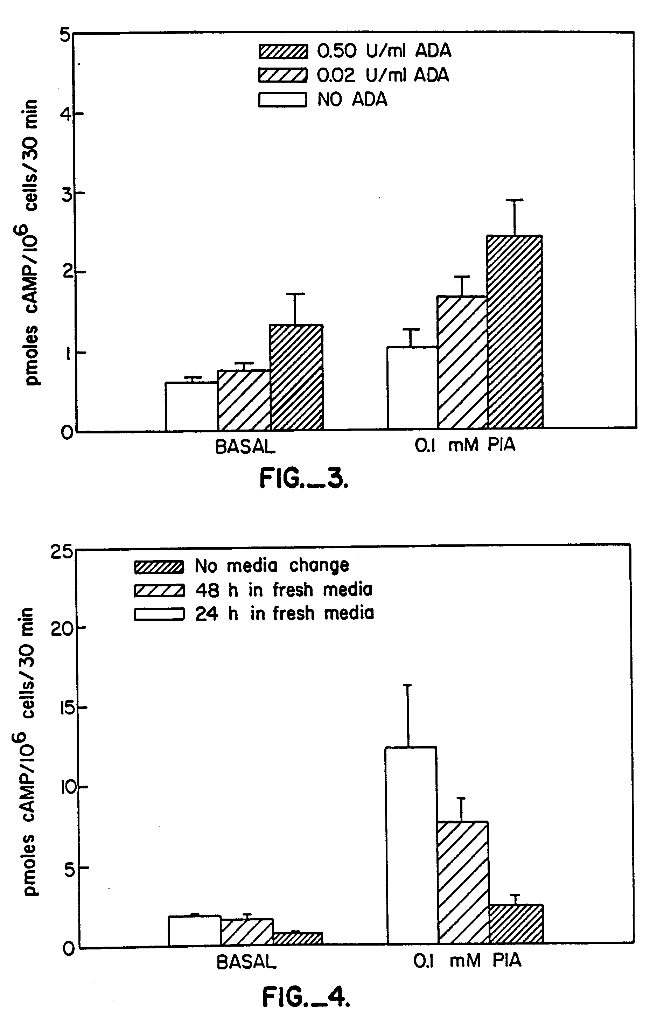

Studies have also been conducted with lymphocytes grown in culture. Several methods to decrease the level of adenosine in the growth media in order to increase the response of cultured lymphocytes to adenosine agonist stimulation were tested. When adenosine deaminase (ADA) is included in the media at concentrations from 0.02 U/ml to 0.5 U/ml, adenosine receptor − stimulated cAMP levels are increased (Fig. 3). Because of the improved response to adenosine stimulation in the presence of ADA, 0.02 U/ml ADA (a concentration found in human serum) can advantageously be added to the culture media. In addition, increased adenosine responsiveness in cells is promoted by changing media 24 and 48 hours prior to assaying adenosine receptor − stimulated cAMP levels (Fig. 4). The cell growth media should not be changed prior to day 6 in culture because loss of various growth promoting factors released by lym − phocytes into the media would limit growth in this critical period.

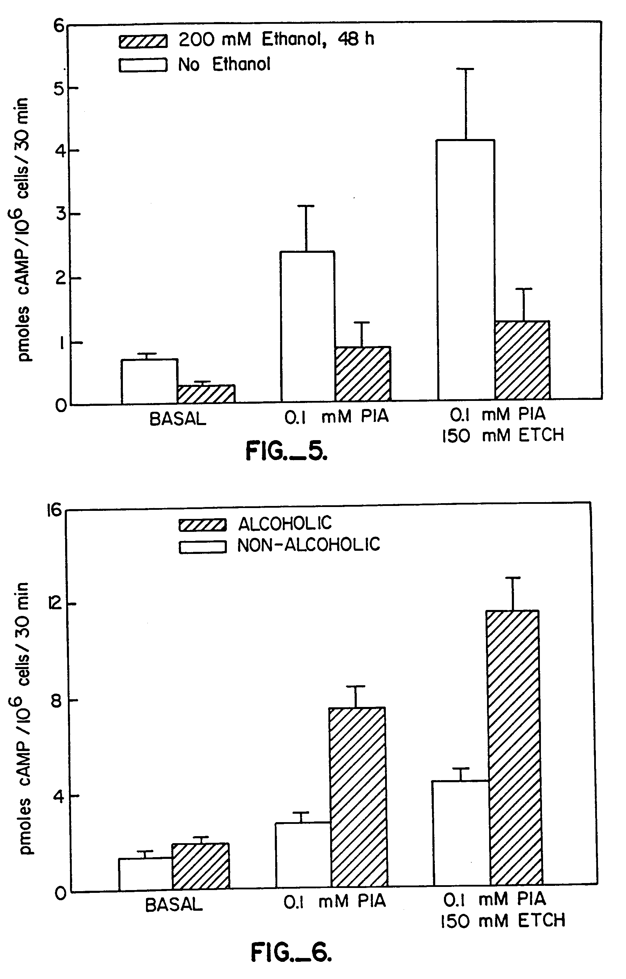

Acute exposure to ethanol further enhances cAMP production stimulated by the adenosine agonist, phenylisopropyladenosine (PIA) in cultured lymphocytes (Fig. 5). This is similar to the effect of ethanol on freshly isolated lymphocytes from normal subjects (Fig. 1). Acute exposure to ethanol does not significantly alter basal levels of cAMP in the cultured cells.

Exposure of lymphocytes from non − alcoholics to 200 mM ethanol for 48 hours in culture (chronic ethanol) results in a decrease in both basal and adenosine receptor − stimulated cAMP production as well as a suppressed response to acute stimulation by ethanol (Fig. 5). A decrease in prostaglandin $E_1$ , ($PGE_1$), receptor − stimulated cAMP levels was also observed. This decrease in cAMP production and resistance to acute stimulation by ethanol is similar to the suppressed responses we have observed in freshly isolated lymphocytes from alcoholics (Fig. 1).

Chronic exposure to ethanol does not alter cell number or the proportion of cells possessing cell surface markers for T − cells, B − cells or the T − cell subpopulations, T − helper and T − suppressor (Table 1). There is also no significant change in the activity of phosphodiesterase after chronic exposure to ethanol. Enzyme activity in lymphocytes cultured with or without ethanol is 19.0 ± 1.9 SEM (n = 4) and 23.7 ± 3.6 pmoles cAMP hydrolyzed/mg prot/min (n = 6), respectively.

TABLE 1

| Lymphocyte Cell Populations | | |
|---|---|---|
| Subpopulation | Cultured Lymphocytes (Day 8) | |
| | Control | 200mM EtOH |
| T − cell | 77 ± 5 | 81 ± 6 |
| B − cell | 9 ± 3 | 8 ± 3 |
| CD4 | 27 ± 8 | 27 ± 11 |
| CD8 | 52 ± 6 | 56 ± 11 |
| Viability | 88 ± 7 | 80 ± 9 |

Cell culture permits examination of the possibility that cells from alcoholics respond differently to chronic exposure to ethanol than cells from non − alcoholics. Lymphocytes from alcoholics were grown for 7 days in culture without ethanol. Adenosine receptor − dependent cAMP accumulation was found to be higher than in cells from non − alcoholics (Fig. 6). This paradoxical increase in adenosine receptor stimulation in lymphocytes from alcoholics after several generations of growth without ethanol might reflect an abnormal homeostatic mechanism in the regulation of cAMP production.

Differences in the sensitivity of lymphocytes from alcoholics compared to non – alcoholics after chronic exposure to ethanol was tested as follows. After exposure of lymphocytes to 100 mM ethanol for 24 hours, lymphocytes from alcoholics showed a significant decrease in adenosine receptor – dependent cAMP production whereas lymphocytes from non – alcoholics showed no change at this concentration of ethanol (Fig. 6).

Taken together, these examples reveal changes in the cAMP signal transduction system in lymphocytes from alcoholics both in the presence and absence of ethanol, despite four or more generations in culture without ethanol. These findings indicate that abnormalities exist in adaptation and recovery of this important signal transduction system which could be unique to cells from alcoholics and could be used as markers for and a genetic predisposition to alcoholism.

Chronic exposure to ethanol causes a heterologous desensitization of several receptors coupled to adenylyl cyclase via $G_s$ (unpublished observations). The function of $G_s$ in cells exposed to 100 mM ethanol for 48 hours was measured in a complementation assay using cells that have no intrinsic $G_s$ activity but which can be reconstituted by exogenously added $G_s$ (Farfel et al. (1980) N. Eng. J. Ned. 303:237 – 242). Cells chronically exposed to ethanol were found to have less $G_s$ activity than control cells. To determine whether this decrease was due to a decrease in the amount of $alpha_s$ (the active subunit of $G_s$) antibodies against $alpha_s$ were used to measure the amount (Towbin et al. (1979) Proc. Natl. Acad. Sci. USA 76:4350) in chronically exposed cells and found decreased amounts in these cells as compared to controls. mRNA for $alpha_s$ was then measured (Sullivan et al. (1986) Proc. Natl. Acad. Sci. USA 83:6687 – 6691) in control cells and cells chronically exposed to ethanol. A decrease in $alpha_s$ mRNA from cells chronically exposed to ethanol was found. Measurements of $alpha_s$ function, amount, and/or mRNA thus appear to be suitable as a biologic test for alcoholism and possibly a genetic marker for susceptibility to alcoholism.

## Claims

1. A method for determining alcoholism susceptibility in a patient, said method comprising:

    isolating cells from the patient;

    culturing the cells in the substantial absence of ethanol for a period sufficient to allow at least three cell divisions;

    stimulating cAMP production in the cultured cells after said sufficient period;

    measuring the level of stimulated cAMP concentration in the cultured cells in the absence of ethanol to obtain a measured value; and

    comparing the measured level with a predetermined normal concentration value, whereby a measured value which is greater than the normal concentration value is diagnostic of alcoholism susceptibility.

2. A method for determining alcoholism susceptibility in a patient, said method comprising:

    isolating cells from the patient;

    culturing the cells in the substantial absence of ethanol for a period sufficient to allow at least three cell divisions;

    stimulating cAMP production in the cultured cells after said sufficient period;

    measuring the level of the stimulated cAMP production in the cultured cells after they have been maintained in the presence of ethanol for at least an additional 24 hours; and

    comparing the measured level with a predetermined normal concentration value, wherein a measured level which is less than the normal concentration value being diagnostic of alcoholism susceptibility.

3. A method according to claim 1 or claim 2 wherein the cAMP concentration is measured in lym – phocytes.

4. A method according to any one of the preceding claims wherein the cells have been cultured in a serum – free media in the absence of alcohol and thereafter exposed to a phosphodiesterase inhibitor.

5. A method according to any one of the preceding claims wherein the cells are stimulated by an adenosine receptor agonist.

8

**6.** A method according to claim 1, wherein the measured value is greater than the normal value by at least about 50%.

**7.** A method according to claim 2, wherein the measured value is less than the normal value by at least about 25%.

**Patentansprüche**

**1.** Verfahren zum Bestimmen der Anfälligkeit eines Patienten gegenüber Alkoholismus, wobei das ge‐ nannte Verfahren umfaßt:
das Isolieren von Zellen vom Patienten;
das Kultivieren der Zellen im wesentlichen in Abwesenheit von Äthanol für einen Zeitraum, der ausreicht, um zumindest drei Zellteilungen zuzulassen;
das Stimulieren der cAMP‐Produktion in den kultivierten Zellen nach dem genannten ausreichenden Zeitraum;
das Messen der Konzentrationshöhe an stimuliertem cAMP in den kultivierten Zellen in Abwesenheit von Äthanol, um einen Meßwert zu erhalten; und
das Vergleichen der gemessenen Höhe mit einem vorherbestimmten normalen Konzentrationswert, wodurch ein Meßwert, der größer als der normale Konzentrationswert ist, eine Diagnose der Anfälligkeit gegenüber Alkoholismus ist.

**2.** Verfahren zum Bestimmen der Anfälligkeit eines Patienten gegenüber Alkoholismus, wobei das ge‐ nannte Verfahren umfaßt:
das Isolieren von Zellen vom Patienten;
das Kultivieren der Zellen im wesentlichen in Abwesenheit von Äthanol für einen Zeitraum, der ausreicht, um zumindest drei Zellteilungen zuzulassen;
das Stimulieren der cAMP‐Produktion in den kultivierten Zellen nach dem genannten ausreichenden Zeitraum;
das Messen der Höhe der Produktion von stimuliertem cAMP in den kultivierten Zellen, nachdem sie zumindest für weitere 24 Stunden in Äthanol gehalten worden sind; und
das Vergleichen der gemessenen Höhe mit einem vorherbestimmten normalen Konzentrationswert, worin eine gemessene Höhe, die geringer als der normale Konzentrationswert ist, eine Diagnose der Anfälligkeit gegenüber Alkoholismus ist.

**3.** Verfahren nach Anspruch 1 oder 2, worin die cAMP‐Konzentration in Lymphozyten gemessen wird.

**4.** Verfahren nach einem der vorhergehenden Ansprüche, worin die Zellen in einem serumfreien Medium in Abwesenheit von Alkohol kultiviert worden und danach einem Phosphodiesteraseinhibitor ausgesetzt worden sind.

**5.** Verfahren nach einem der vorhergehenden Ansprüche, worin die Zellen durch einen Adenosinrezepto‐ ragonisten stimuliert werden.

**6.** Verfahren nach Anspruch 1, worin der gemessene Wert um zumindest etwa 50% höher als der normale Wert ist.

**7.** Verfahren nach Anspruch 2, worin der gemessene Wert um zumindest etwa 25% niedriger als der normale Wert ist.

**Revendications**

**1.** Méthode pour déterminer la prédisposition à l'alcoolisme chez un patient, ladite méthode consistant à :
isoler des cellules du patient ;
mettre les cellules en culture en l'absence sensible d'éthanol pendant une période de temps suffisante pour permettre au moins trois divisions des cellules ;
stimuler la production de cAMP dans les cellules en culture après ladite période suffisante de temps ;
mesurer le niveau de la concentration en cAMP stimulé dans les cellules en culture en l'absence d'éthanol pour obtenir une valeur mesurée ; et

9

comparer le niveau mesuré avec une valeur normale prédéterminée de concentration, une valeur mesurée qui est plus importante que la valeur de concentration normale étant le diagnostic d'une prédisposition à l'alcoolisme.

2. Méthode pour déterminer une prédisposition à l'alcoolisme chez un patient, ladite méthode consistant à :

isoler des cellules du patient ;

mettre les cellules en culture en l'absence sensible d'éthanol pendant une période de temps suffisante pour permettre au moins trois divisions des cellules ;

stimuler la production de cAMP dans les cellules en culture après ladite période suffisante de temps ;

mesurer le niveau de la production de cAMP stimulé dans les cellules en culture après les avoir maintenues en présence d'éthanol pendant au moins 24 heures supplémentaires ; et

comparer le niveau mesuré à une valeur normale prédéterminée de concentration, un niveau mesuré qui est plus faible que la valeur normale de concentration étant le diagnostic d'une prédisposition à l'alcoolisme.

3. Méthode selon la revendication 1 ou la revendication 2, où la concentration en cAMP est mesurée dans les lymphocites.

4. Méthode selon l'une quelconque des revendications précédentes, où les cellules ont été mises en culture dans un milieu sans sérum en l'absence d'alcool et ensuite exposées à un inhibiteur de phosphodiestérase.

5. Méthode selon l'une quelconque des revendications précédentes, où les cellules sont stimulées par un agoniste du récepteur d'adénosine.

6. Méthodes selon la revendication 1, où la valeur mesurée est plus importante que la valeur normale d'au moins environ 50%.

7. Méthode selon la revendication 2, où la valeur mesurée est plus faible que la valeur normale d'au moins environ 25%.

FIG._1.

FIG._2.

FIG._3.

FIG._4.

FIG._5.

FIG._6.

FIG._7.